(19) **Europäisches Patentamt**
**European Patent Office**
**Office européen des brevets**

(11) **EP 2 173 242 B1**

(12) # EUROPEAN PATENT SPECIFICATION

(45) Date of publication and mention
of the grant of the patent:
**04.05.2011 Bulletin 2011/18**

(51) Int Cl.:
*A61B 5/024* (2006.01)    *A61B 5/08* (2006.01)
*A61B 5/00* (2006.01)    *G01R 23/16* (2006.01)

(21) Application number: **08762370.8**

(22) Date of filing: **13.06.2008**

(86) International application number:
**PCT/GB2008/002043**

(87) International publication number:
**WO 2009/016334 (05.02.2009 Gazette 2009/06)**

(54) **METHOD AND APPARATUS FOR MEASURING BREATHING RATE**

VERFAHREN UND GERÄT ZUR MESSUNG DER ATEMFREQUENZ

PROCÉDÉ ET APPAREIL DE MESURE DU RYTHME RESPIRATOIRE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **30.07.2007 GB 0714807**

(43) Date of publication of application:
**14.04.2010 Bulletin 2010/15**

(73) Proprietor: **Oxford Biosignals Limited
Thame, Oxfordshire OX9 3EZ (GB)**

(72) Inventors:
• **TARASSENKO, Lionel
Parks Road, Oxford OX1 3PJ (GB)**
• **FLEMING, Susannah
Abingdon, Oxon OX14 4SE (GB)**

(74) Representative: **Nicholls, Michael John
J.A. Kemp & Co.
14 South Square
Gray's Inn
London
WC1R 5JJ (GB)**

(56) References cited:
WO-A-00/21438        WO-A-03/005893
WO-A-03/051198        WO-A-03/071938
WO-A-2004/075746

• LISA LAZARECK ET AL: "Detection of apnoeic
and breathing activity through pole-zero analysis
of the SpO2 signal" ENGINEERING IN MEDICINE
AND BIOLOGY SOCIETY, 2006. EMBS '06. 28TH
ANNUAL INTERNATIONAL CONFERENCE OF
THE IEEE, IEEE, PI, 30 August 2006 (2006-08-30),
pages 3879-3882, XP031236370 ISBN:
978-1-4244-0032-4
• SG FLEMING ET AL: "A comparison of signal
processing techniques for the extraction of
breathing rate from the photoplethysmogram"
PROCEEDINGS OF WORLD ACADEMY OF
SCIENCE, ENGINEERING AND TECHNOLOGY,
WORLD ACADEMY OF SCIENCE, ENGINEERING
AND TECHNOLOGY, TR, vol. 24, 1 October 2007
(2007-10-01), pages 276-280, XP009105542 ISSN:
1307-6884

EP 2 173 242 B1

**Description**

The present invention relates to a method and apparatus for measuring the breathing rate of a human or animal subject.

Measurement of the breathing rate, also known as respiratory rate, is an important aspect of health monitoring, particularly in subjects who are at risk from respiratory suppression. However existing methods of measuring breathing rate have a number of difficulties associated with them. For example, electrical impedance pneumography, which is based on the measurement of the change in electrical impedance across the chest between inspiration and expiration, is prone to artefact, particularly when a patient moves. Airflow sensors, for instance nasal thermistors, which measure the breathing rate by monitoring the difference in temperature between inhaled and exhaled air, are uncomfortable for patients to use over a long period of time. Acoustic monitoring of the throat has also been proposed, but again this is subject to artefact.

Proposals have been made for deriving a breathing rate signal from the photoplethysmogram (hereinafter referred to as PPG), this being a signal normally used to measure oxygen saturation in the blood and the heart rate (also referred to as the pulse rate). The PPG is obtained by measuring the changes in the absorption of light at one of two wavelengths (usually red and infra-red) caused by the flow of arterial blood in a body segment such as the finger or earlobe. The PPG signal is an oscillatory signal at the heart rate, but with a longer period modulation at the breathing rate. Extracting the respiratory waveform from the PPG is not easy and three categories of techniques have been proposed: digital filtering, frequency-domain (periodogram) analysis and time-frequency (wavelet) analysis. To date, though, these techniques have not proved entirely satisfactory, mainly because of the variability of the respiratory modulation.

WO-A-1-00/21438, on which the pre-characterising portion of claim 1 is based, WO-A2-2004/075746 and WO-A1-03/071938 disclose methods for analysing photoplethysmograms to determine respiration rate. WO-A1-03/051198 discloses a method of combining two different breathing rate signals, including, for example, one from a photoplethysmogram, to obtain a better estimate of breathing rate.

Accordingly the present invention provides a method of measuring the breathing rate of a subject, comprising obtaining a photoplethysmogram from the subject, and further comprising the steps of modelling each of a plurality of discrete time periods of the photoplethysmogram using respective autoregressive models, finding poles of the autoregressive models of the discrete time periods of the photoplethysmogram, and calculating from the poles for each period the breathing rate for each time period.

Thus the present invention uses autoregressive (AR) modelling of successive sections or windows of the PPG waveform to measure the frequency content in each section. Each pole of the AR model represents a particular frequency component, and the breathing rate is obtained by identifying the pole which represents the respiratory waveform. Finding this pole in each successive section of the signal allows calculation of the breathing rate for each of the successive sections.

Preferably the discrete time periods are successive overlapping windows. The amount of overlap and the length of window can be chosen according to the amount of delay acceptable before outputting the breathing rate for a particular time period and depending on the level of accuracy required (longer time periods allow greater accuracy of the autoregressive modelling, but require a longer wait before the result is output). For example the windows may be successive windows from 20 seconds to 1 minute long, each displaced from the other by 5 to 15 seconds. In one example the windows are 30 seconds long each being displaced by 5 seconds. This results in output of a breathing rate every 5 seconds, this corresponding to the average breathing rate over the preceding 30 seconds.

Preferably the PPG signal is downsampled before it is modelled. Downsampling allows the poles corresponding to the breathing rate to be identified more easily. The PPG signal, typically obtained at a sampling frequency of 100 Hz, may, for example, be downsampled to 1Hz or 2Hz, this being effective to attenuate the heart rate component which can have subharmonics at similar frequencies to the breathing rate. Preferably the (downsampled) PPG signal is also detrended to remove any DC offset. This simplifies the modelling process. The PPG signal may also be low-pass filtered prior to downsampling to attenuate frequency components at the heart rate frequency and above.

To identify the pole corresponding to the breathing rate in the AR model, poles are identified which correspond to frequencies in a range of interest, e.g. between 0.08 and 0.7 Hz (4.8 - 42 breaths per minute), and the pole with the lowest frequency in this range whose magnitude is more than a predetermined threshold is identified as the dominant pole representing the breathing rate. This avoids, for example, selecting poles representing harmonics of the breathing rate (e.g. at double the breathing rate). The magnitude of the threshold may be set at 95% of the pole with the greatest magnitude amongst those in the frequency range of interest.

The order of the autoregressive model is preferably from 7 to 13, more preferably 9, 10 or 11. The higher the order of the model the more accurate the modelling, but the greater the processing time and the greater the number of poles from which the breathing rate pole needs to be identified.

The method above produces a series of breathing rate measurements, one for each window. Preferably this series of measurements is further processed to improve the breathing rate estimate. For example, because it would be unusual for the breathing rate to change greatly from one reading to the next, the series of measurements may be smoothed, e.g. using a Kalman filter. This is effective to reject any outlier measurement outside a predetermined change in breathing rate.

The breathing rate measurements produced by the measurements above may also be combined with breathing rate measurements obtained from another sensor, for example, impedence pneumography, nasal thermistor, etc., and the two measurements may be combined with respective weights derived from the level of confidence in each of the measurements as described in WO 03/051198.

Another aspect of the invention provides apparatus for measuring the breathing rate in accordance with the method above. Such apparatus accepts the PPG input and, optionally, a breathing rate input by another sensor, processes the signals as described above and displays the breathing rate on a display.

The invention may be embodied in software and thus extends to a computer program comprising program code means for executing the processing steps in the method, and to a computer-readable storage medium carrying the program.

The invention will be further described by way of example with reference to the accompanying drawings in which:-

Figure 1 schematically illustrates a breathing rate (BR) measurement apparatus according to one embodiment of the present invention;

Figure 2 is a flow diagram of the processing according to an embodiment of the present invention;

Figure 3(a), (b) and (c) illustrate respectively the raw PPG signal, downsampled and detrended PPG signals and Figure 3(d) illustrates the poles of the AR model calculated therefrom using an 11th order AR model with a downsampled frequency of 1Hz.

Figure 4(a), (b), (c) and (d) show respectively the raw PPG signal, low-pass filtered, downsampled and de-trended PPG signals and Figure 4(e) shows the poles of the AR models calculated therefrom using a 9th order AR model and a downsampled frequency of 2Hz;

Figure 5(a) illustrates a comparison of the breathing rate calculated using an embodiment of the invention with a known reference breathing rate, illustrating rejection of breathing rate values and Figure 5(b) illustrates the breathing rate measurements and rejection limits used in one embodiment of the invention.

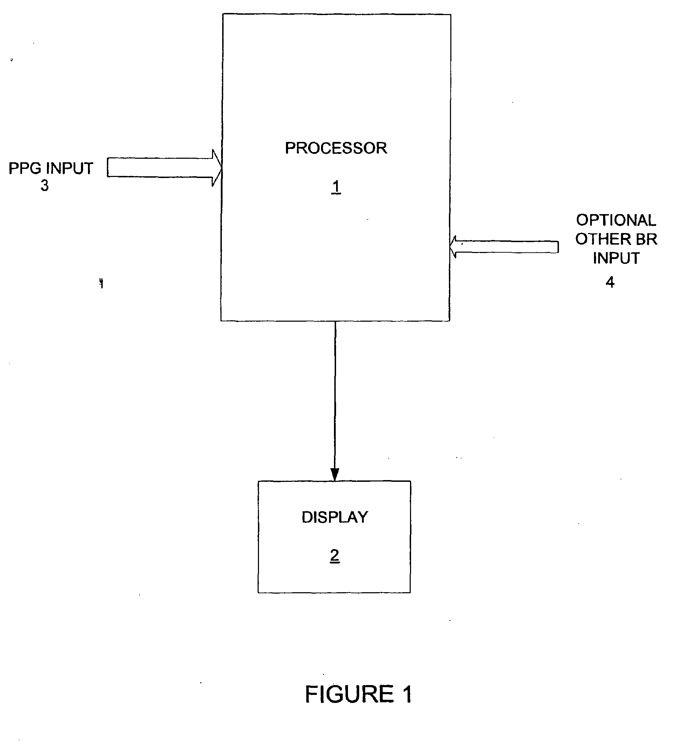

Figure 1 schematically illustrates a breathing rate measurement apparatus in accordance with one embodiment of the invention. The modelling and filtering processes which produce the breathing rate measurement are conducted in a processor 1 and a breathing rate is displayed on a display 2 as a number or a time series or both. The processor 1 receives a PPG input 3 from a standard PPG sensor used for oxygen saturation measurement and heart rate measurement and optionally another breathing rate input 4 from a different type of sensor such as a nasal thermistor and an electrical impedance pneumograph.

Before describing the signal processing in this embodiment in detail it may be useful hereto give a brief explanation of the general principles of autoregressive (AR) modelling, though AR modelling is well-known, for example, in the field of speech analysis.

AR modelling can be formulated as a linear prediction problem where the current value $x(n)$ of the signal can be modelled as a linearly weighted sum of the preceding p values. Parameter $p$ is the model order, which is usually much smaller than the length $N$ of the sequence of values forming the signal. Thus:-

$$x(n) = -\sum_{k=1}^{p} a_k x(n-k) + e(n) \qquad (1)$$

The value of the output $x(n)$ is therefore a linear regression on itself, with an error $e(n)$, which is assumed to be normally distributed with zero mean and a variance of $\sigma^2$. The model can alternatively be visualised in terms of a system with input $e(n)$, and output $x(n)$, in which case the transfer function $H$ can be formulated as shown below:

$$H(z) = \frac{1}{\sum_{k=1}^{p} a_k z^{-k}} = \frac{z^p}{(z-z_1)(z-z_2)...(z-z_p)} \qquad (2)$$

As shown in Equation 2, the denominator of $H(z)$ can be factorised into $p$ terms. Each of these terms defines a root $z_i$ of the denominator of $H(z)$, corresponding to a pole of $H(z)$. Since $H(z)$ has no finite zeros, the AR model is an all-pole model. The poles occur in complex-conjugate pairs and define spectral peaks in the power spectrum of the signal. They can be visualised in the complex plane as having a magnitude (distance from the origin) and phase angle (angle with the real axis). Higher magnitude poles corresponding to higher magnitude spectral peaks and the resonant frequency of each spectral peak is given by the phase angle of the corresponding pole. The phase angle θ corresponding to a given frequency f, is defined by Equation 3 which shows that it is also dependent on the sampling interval $\Delta t$ (reciprocal of the sampling frequency):

$$\theta = 2\pi f \Delta t \qquad (3)$$

Thus fitting a suitable order AR model to a signal reveals the spectral composition of the signal. As will be explained below, the pole in an AR model of the PPG signal which corresponds to the breathing rate can be identified from a search of the poles with phase angles within a range defined by the expected breathing frequencies for a normal signal.

To find the poles, the model parameters $a_k$ are first obtained using the Yule-Walker equations to fit the model to the signal and from the values of $a_k$ the values of the $p$ poles $z_l$ to $z_p$ can be calculated. The $p$ poles of $H(z)$, which correspond to the $p$ roots $z_i$ ($i$ = 1 to p) of the denominator of $H(z)$ are found using standard mathematical procedures (for example, the MATLAB routine *roots*). As each pole $z_k$ can be written as a complex number $x_k + jy_k$, the frequency represented by that pole can be calculated from the phase angle of that pole in the upper half of the complex plane:

$$\theta = \tan^{-1} y/x = 2\pi f_k . 1/f_s \qquad (4)$$

where $f_s$ is the sampling frequency and the magnitude $r$ is $(x^2+y^2)^{1/2}$.

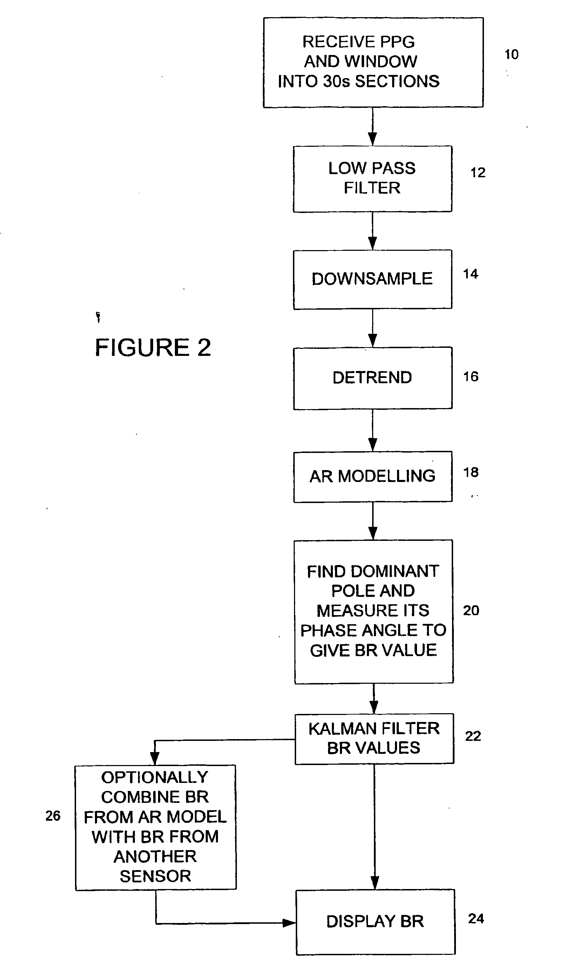

With that background in mind, Figure 2 illustrates the processing of the PPG signal in accordance with one embodiment of the invention. The PPG signal is received in step 10, this typically being a signal sampled at a high sampling frequency $f_s$ such as 100Hz. The raw PPG signal is divided into discrete overlapping time periods or windows each, for example, 30 seconds long and displaced from its predecessor by 5 seconds, though different window sizes and displacements can be used if desired. Each breathing rate measurement will be, in essence, an average over the window, so although the use of a longer window can increase the precision of the modelling, it also introduces a longer delay and means that the measurement obtained is less representative of the breathing rate at the current instant.

The PPG signal is prefiltered using a low pass filter in step 12 and is downsampled in step 14 as will be explained below.

The use of short, 30 second, windows reduces the amount of data available for estimating the parameters of the AR model and so the accuracy of the breathing rate measurement will be degraded. Although the amount of data can be increased by reducing the downsampling ratio, the heart rate frequency would again be more prominent in the downsampled signal and poles due to subharmonics of the heart rate could also appear at similar frequencies to the breathing rate. To allow a lower downsampling ratio, therefore, in this embodiment of the invention pre-filtering of the PPG signal is carried out using an FIR low pass filter as illustrated at step 12. In this embodiment the filter uses the Kaiser windowing function with a transition band from 0.4 to 0.8 Hz (24 to 48 cycles per minute). The upper frequency is low enough to ensure that most of the heart rate frequency information is removed (since the heart rate is usually 60 beats/min or above) without introducing excessive attenuation of the breathing rate information. The pass-band ripple was designed to be 5% and the stop band attenuation was designed to be 30 dB. It should be noted that such pre-filtering is optional, and if a higher downsampling ratio is used, such pre-filtering may not be needed.

At the typically high sampling rates of the PPG signal, the phase angles corresponding to breathing frequencies would be very small which would make it impossible to identify the breathing pole (since it would be likely to be subsumed into the real axis or DC pole). It is therefore necessary to downsample the signal to increase the angular resolution of the low frequency information. The downsampling ratio is chosen so as to ensure that the cardiac-synchronous pulsatile component of the PPG is no longer dominant and hence that the main poles of the AR model do not model the heart rate information, rather than the breathing rate information.

To improve the stability and accuracy of the AR model, it is also preferable to remove any DC offset from the downsampled PPG signal by detrending in step 16.

The processed 30 second section of PPG signal is then modelled using AR modelling in step 18 to find the $p$ poles in accordance with Equation 2. In step 20 the dominant pole representing the breathing rate is then found as explained below, and the frequency (breathing rate) that it represents is calculated using Equation 4.

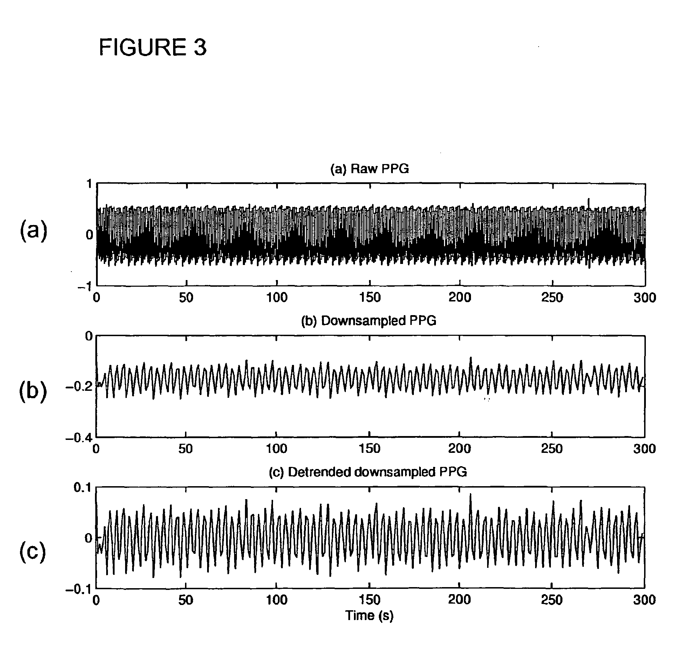

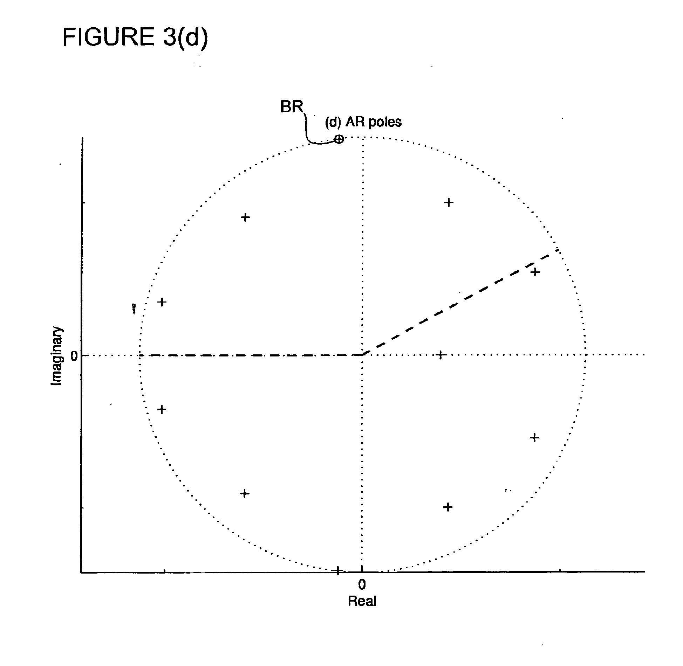

Figure 3(d) illustrates the poles obtained using an 11th order AR model with a downsampling frequency of 1 Hz on the 30 second section of PPG signal illustrated (in this case without the low pass filtering of step 12). As can be seen each of the poles is one of a complex conjugate pair and so to identify the pole corresponding to the breathing rate it is necessary only to consider the upper half of the diagram. To identify the pole representing breathing rate both the magnitude of the pole (distance r from the origin) and frequency (phase angle θ) are considered. The pole representing the breathing frequency should have a high magnitude and be in the region of 4.8 - 42 breaths per minute. Therefore in the diagram a sector of interest (indicated by broken radial lines) is defined from 0.08 to 0.7 Hz (4.8 to 42 cycles per minute) and the lowest frequency pole within that sector that has a magnitude of more than 95% of the pole with the highest magnitude within the same sector is identified (labelled BR in the diagram). In Figure 3(d) the phase angle of the breathing pole labelled BR corresponds to a breathing frequency of 0.27 Hz (16 breaths per minute).

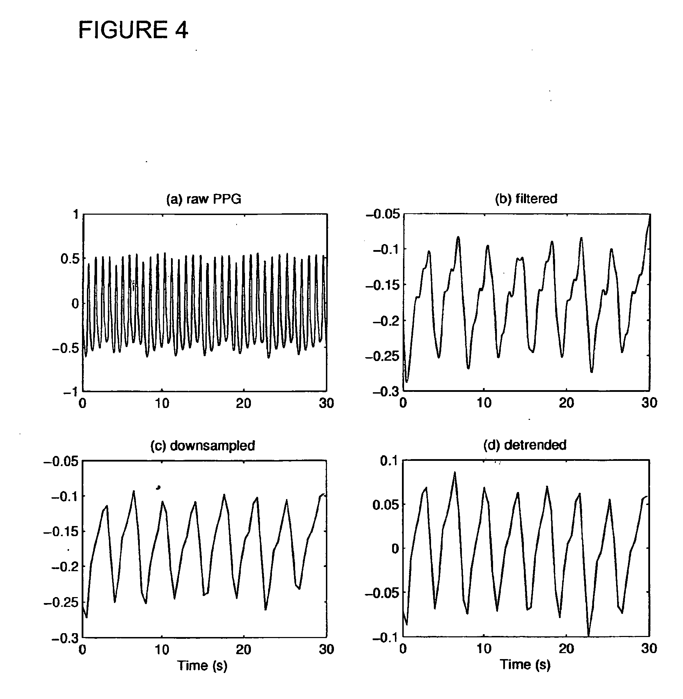

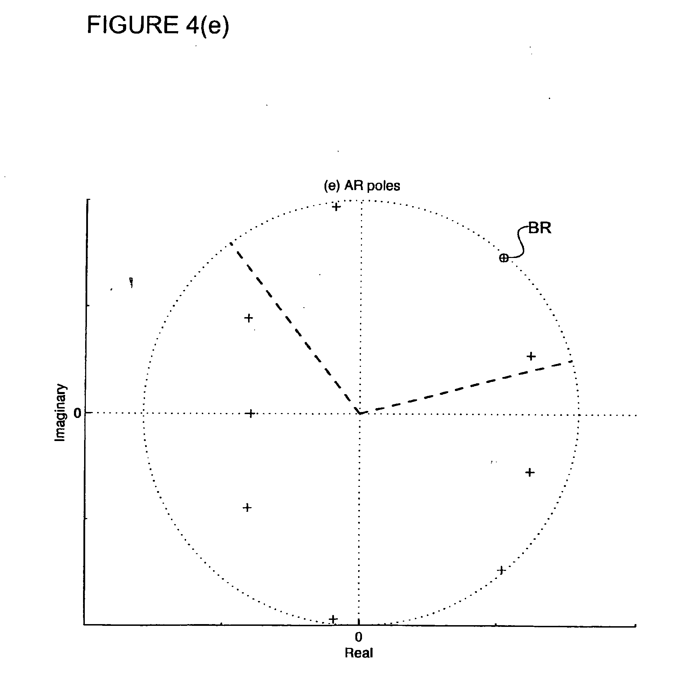

Figure 4(e) illustrates similar results for a 9th order AR model with a downsampling frequency of 2 Hz. In this case because the downsampling ratio is lower, the low pass filtering of step 12 is used. The phase angle of the breathing pole BR corresponds to a breathing frequency of 0.26 Hz (16 breaths per minute).

Although the method above gives a good estimate of the breathing rate, which could be directly displayed in step 24, further steps can be taken to reduce the effect of artefacts in the PPG signal. This is particularly important if short length windows are used. In particular artefacts and other similar signal quality problems can lead into sudden changes in the estimated breathing rate and reductions in the magnitude of the breathing pole. In this embodiment of the invention, therefore, a Kalman filter is used in step 22 to smooth the time series of breathing rate measurements and reject those representing too large a change in breathing rate or pole magnitude.

As is well-known, a Kalman filter uses probabilistic reasoning to estimate the state **x** of a system based on measurements z. In this case, a 2-dimensional Kalman filter is used, with **x** and z each being two-element vectors (with elements corresponding to the breathing rate and breathing pole magnitude. The evolution of these vectors is described by Equations 5.1 and 5.2 below.

$$\mathbf{x}_i = \mathbf{A}\mathbf{x}_{i-1} + \mathbf{w}$$
$$\mathbf{w} \approx N(0, \mathbf{Q}) \qquad (5.1)$$

The square matrix **A** describes how state **x** evolves over discrete time intervals (i-1 is followed by i), and is known as the "state transition matrix". In this case we do not expect a particular trend in either breathing rate or pole magnitude over time, so **A** is the identity matrix. The vector **w** is the "process noise" describing the noise in the true state, and is assumed to be normally distributed with zero mean and covariance matrix **Q**. Since the breathing rate and pole magnitude can be expected to be independent, **Q** in this case is a diagonal matrix, with no covariance terms.

$$\mathbf{z}_i = \mathbf{H}\mathbf{x}_i + \mathbf{v}$$
$$\mathbf{v} \approx N(0, \mathbf{R}) \qquad (5.2)$$

The current value of observation z is assumed to be a function of the current state **x** with added noise v. The square matrix **H** describes how the measurement relates to the state, and is known as the "observation matrix". In this case, we measure the two states directly, so **H** is the identity matrix. The "measurement noise", v, is assumed to be normally distributed with zero mean and covariance matrix **R**. As with the process noise, **R** in this case is a diagonal matrix with no covariance terms.

Before running the filter, it is necessary to supply initial values for the state estimate $X_0$ and its covariance matrix $\mathbf{P}_0$. The filter first estimates the new values of **x** and **P** using Equations 5.3 and 5.4.

$$\mathbf{x}_i = \mathbf{A}\mathbf{x}_{i-1} \qquad (5.3)$$

$$P_i = AP_{i-1}A^T + Q \quad (5.4)$$

From these, the Kalman gain **K** and the innovation **I** can be calculated using Equations 5.5 and 5.6. These will be used to update the prediction of the state **x** using the current value of the measurement **z**.

$$K_i = P_iH(HP_iH^T + R)^{-1} \quad (5.5)$$

$$I_i = z_i - Hx_i \quad (5.6)$$

**x** and **P** should only be updated using the latest measurement **z** if the innovation (the difference between the measurement expected for the estimated state and the actual measurement) is sufficiently low. If the innovation is within defined bounds the update is carried out using Equations 5.7 and 5.8. If the innovation is too great, the measurement is flagged as invalid and no update is carried out.

$$x_i = x_i + KI_i \quad (5.7)$$

$$P_i = P_i - K_iHP_i \quad (5.8)$$

The values of the matrices defining the Kalman filter used for this application are shown below. The state is initialised to a value of 0.95 for the breathing pole magnitude, and to the mean of the first three measurements of breathing for the breathing rate. Since **A**, **H**, **Q**, **R** and **P**$_0$ are all diagonal, the two states are uncoupled, and the filter behaves in the same way as two one-dimensional filters.

$$\mathbf{z} = \begin{bmatrix} polemagnitude \\ breathingrate \end{bmatrix} \quad \mathbf{x}_0 = \begin{bmatrix} 0.95 \\ mean(z_1, z_2, z_3) \end{bmatrix} \quad \mathbf{P}_0 = \begin{bmatrix} 1 \times 10^{-3} & 0 \\ 0 & 1 \end{bmatrix}$$

$$\mathbf{A} = \mathbf{H} = \begin{bmatrix} 1 & 0 \\ 0 & 1 \end{bmatrix} \quad \mathbf{Q} = \begin{bmatrix} 1 \times 10^{-5} & 0 \\ 0 & 0.05 \end{bmatrix} \quad \mathbf{R} = \begin{bmatrix} 1 \times 10^{-4} & 0 \\ 0 & 0.2 \end{bmatrix}$$

The measurement is considered valid, and the state and variance are updated, if both of the following conditions are satisfied:

1. The pole magnitude innovation is greater than $-7\sqrt{P_i(1,1)}$, which corresponds to ensuring that the measurement is not more than 7 standard deviations from the expected state. Only deviations in the negative direction are penalised, as large increases in pole magnitude should correspond to improved accuracy.

2. The absolute value of the breathing rate innovation is less than $3\sqrt{P_i(2,2)}$, which corresponds to ensuring that the measurement lies within 3 standard deviations of the expected state. Deviations in both directions are penalised equally.

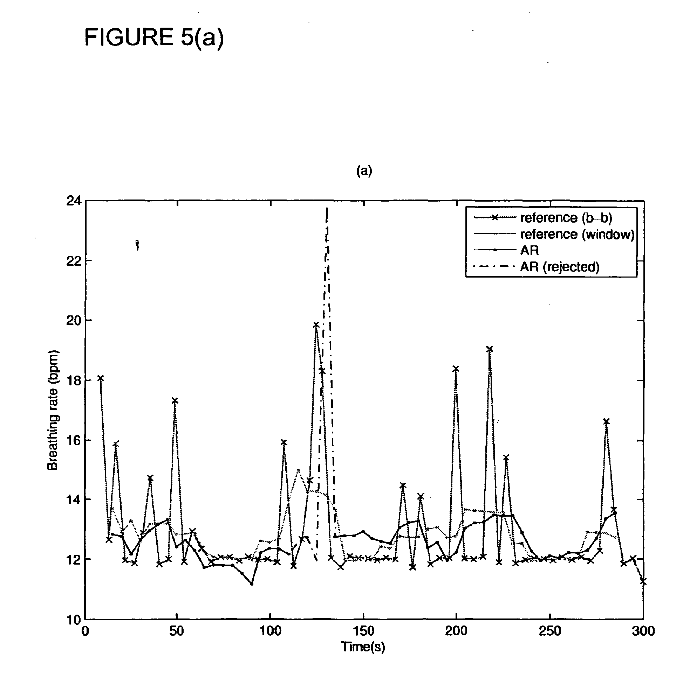

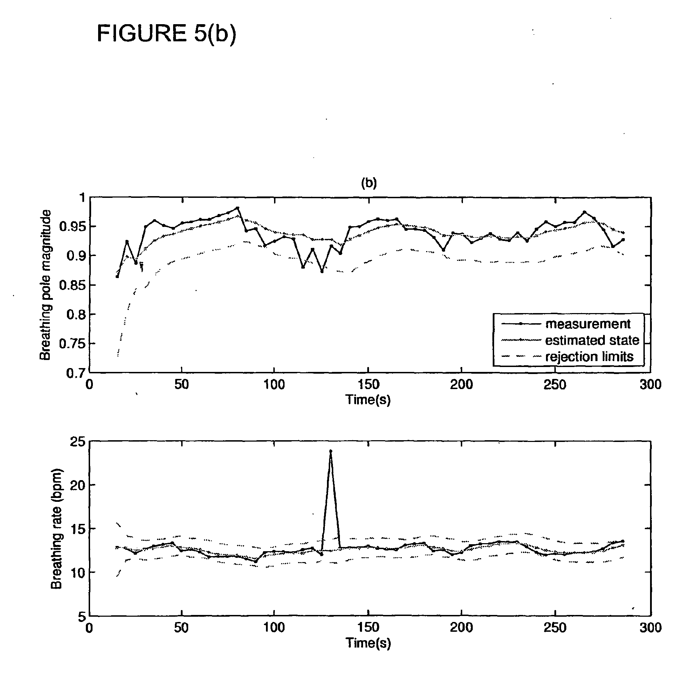

Figure 5 illustrates the effect of using the Kalman filter to reject inaccurate or artefactual measurements. Figure 5(a) illustrates the reference breathing rate and the breathing rate estimated using the AR model and shows dotted a rejected breathing rate estimate. Figure 5(b) shows the estimated states from the Kalman filter for the breathing pole magnitude and the breathing rate, together with the rejection limits dotted and it can be seen that the artefact at about 130 seconds clearly exceeds the rejection limit.

In some circumstances the accuracy of the AR breathing pole may be low. In order to allow estimation of the breathing rate even in such circumstances the AR breathing rate estimate may be fused in step 26 with an estimate from another breathing rate sensor, for example a conventional sensor. The two measurements may be fused using the technique described in WO 03/051198 which uses a one-dimensional Kalman filter on each series of breathing rate measurements to calculate a confidence value associated with that measurement, this confidence value then being used as a weight in combining the two breathing rate estimates.

## Claims

1. A method of measuring the breathing rate of a subject, comprising obtaining (10) a photoplethysmogram from the subject, and **characterised by** further comprising the steps of: modelling (18) each of a plurality of discrete time periods of the photoplethysmogram using respective autoregressive models, finding (20) poles of the autoregressive models of the discrete time periods of the photoplethysmogram, and calculating (20) from the poles for each period the breathing rate for each time period.

2. A method according to claim 1 wherein the time periods are successive overlapping windows.

3. A method according to claim 2 wherein the successive overlapping windows are displaced from each other by 5 to 15 seconds and are from 20 secs to 1 minutes long, more preferably displaced from each other by 5 seconds and are 30 seconds long.

4. A method according to any one of the preceding claims further comprising the step of downsampling (14) and/or detrending (16) the photoplethysmogram before modelling.

5. A method according to any one of the preceding claims further comprising the step of low-pass filtering (12) the photoplethysmogram before modelling.

6. A method according to any one of the preceding claims further comprising the step of identifying as a pole representing the breathing rate a pole (BR) which has a magnitude greater than a predetermined threshold.

7. A method according to any one of the preceding claims further comprising the step of identifying as a pole representing the breathing rate a pole (BR) which corresponds to a predefined allowable range of breathing rates.

8. A method according to any one of the preceding claims wherein the order of the model is from 7 to 13.

9. A method according to any one of the preceding claims wherein the order of the model is 9, 10 or 11.

10. A method according to any one of the preceding claims further comprising the step of filtering (22) the breathing rate measurements for the plurality of discrete time periods to produce a smoothed breathing rate measurement.

11. A method according to claim 10 wherein said filtering step (22) comprises Kalman filtering.

12. A method according to claim 10 or 11 further comprising rejecting measurements which represent greater than a predetermined change in breathing rate.

13. A method according to any one of the preceding claims further comprising the step of combining (26) the measurement of breathing rate with a breathing rate measurement from another sensor.

14. A method according to claim 13 wherein the combination is a weighted combination with weights derived from confidence in the measurements.

15. Apparatus for measuring the breathing rate of a subject, comprising an input (3) for receiving a photoplethysmogram from the subject, a data processor (1) configured to execute the steps of modelling (18) each of a plurality of discrete time periods of the photoplethysmogram using respective autoregressive models, finding the poles (20) of the autoregressive models of the discrete time periods of the photoplethysmogram, and calculating (20) from the poles for each period the breathing rate for each time period as defined in any one of claims 1 to 14.

**Patentansprüche**

1. Verfahren zur Messung der Atemfrequenz eines Subjekts, umfassend das Erhalten (10) eines Photoplethysmogramms von dem Subjekt, und **dadurch gekennzeichnet, dass** es außerdem folgende Schritte umfasst: Modellierung (18) jedes aus einer Vielzahl von unterschiedlichen Zeiträumen des Photoplethysmogramms unter Verwendung entsprechender autoregressiver Modelle, Auffindung (20) der Pole der autoregressiven Modelle der unterschiedlichen Zeiträume des Photoplethysmogramms, und Berrechnung (20) aus den Polen für jeden Zeitraum der Atemfrequenz für jeden Zeitraum.

2. Verfahren nach Anspruch 1, worin die Zeiträume sukzessive überlappende Fenster sind.

3. Verfahren nach Anspruch 2, worin die sukzessiven überlappenden Fenster um 5 bis 15 Sekunden gegeneinander versetzt sind und von 20 Sekunden bis 1 Minute lang sind, bevorzugter um 5 Sekunden gegeneinander versetzt sind und 30 Sekunden lang sind.

4. Verfahren nach jedem der vorangehenden Ansprüche, außerdem umfassend den Schritt des Downsampling (Heruntertakten) (14) und/oder Detrending (Trendbereinigen) (16) des Photoplethysmogramms vor der Modellierung.

5. Verfahren nach jedem der vorangehenden Ansprüche, außerdem umfassend den Schritt des Tiefpassfilterns (12) des Photoplethysmogramms vor der Modellierung.

6. Verfahren nach jedem der vorangehenden Ansprüche, außerdem umfassend den Schritt des Identifizierens als einem Pol, der die Atemfrequenz darstellt, eines Pols (BR), der einen größeren Wert als ein zuvor bestimmter Grenzwert aufweist.

7. Verfahren nach jedem der vorangehenden Ansprüche, außerdem umfassend den Schritt des Identifizierens als einem Pol, der die Atemfrequenz darstellt, eines Pols (BR), der einem zuvor definierten zulässigen Bereich von Atemfrequenzen entspricht.

8. Verfahren nach jedem der vorangehenden Ansprüche, worin die Ordnung des Modells von 7 bis 13 ist.

9. Verfahren nach jedem der vorangehenden Ansprüche, worin die Ordnung des Modells 9, 10 oder 11 ist.

10. Verfahren nach jedem der vorangehenden Ansprüche, außerdem umfassend den Schritt des Filterns (22) der Atemfrequenz-Messungen für die Vielzahl der unterschiedlichen Zeiträume, um eine geglättete Atemfrequenz-Messung zu erzeugen.

11. Verfahren nach Anspruch 10, worin der Filterschritt (22) eine Kalman-Filterung umfasst.

12. Verfahren nach Anspruch 10 oder 11, außerdem umfassend das Verwerfen von Messungen, die eine größere als eine zuvor bestimmte Veränderung in der Atemfrequenz darstellen.

13. Verfahren nach jedem der vorangehenden Ansprüche, außerdem umfassend den Schritt des Kombinierens (26) der Atemfrequenz-Messung mit einer Atemfrequenz-Messung von einem anderen Sensor.

14. Verfahren nach Anspruch 13, worin die Kombination eine gewichtete Kombination mit Gewichtungen ist, die von dem Konfidenzniveau in den Messungen hergeleitet sind.

15. Gerät zur Messung der Atemfrequenz eines Subjekts, umfassend eine Eingabe (3) für den Erhalt eines Photoplethysmogramms von dem Subjekt, einen Datenprozessor (1), der zur Ausführung der Schritte der Modellierung (18) jedes aus einer Vielzahl von unterschiedlichen Zeiträumen des Photoplethysmogramms unter Verwendung entsprechender autoregressiver Modelle, Auffindung der Pole (20) der autoregressiven Modelle der unterschiedlichen Zeiträume des Photoplethysmogramms, und Berrechnung (20) aus den Polen für jeden Zeitraum der Atemfrequenz für jeden Zeitraum, wie in jedem der Ansprüche 1 bis 14 definiert, konfiguriert ist.

**EP 2 173 242 B1**

**Revendications**

1. Procédé de mesure du rythme respiratoire d'un sujet, consistant à obtenir (10) un photopléthysmogramme du sujet, et **caractérisé en ce qu'**il comprend en outre les étapes consistant à : modeler (18) chacune d'une pluralité de périodes de temps discrètes du photopléthysmogramme en utilisant les modèles autorégressifs respectifs, trouver (20) les pôles des modèles autorégressifs des périodes de temps discrètes du photopléthysmogramme, et calculer (20) à partir des pôles de chaque période le rythme respiratoire pour chaque période de temps.

2. Procédé selon la revendication 1, dans lequel les périodes de temps sont des fenêtres chevauchantes successives.

3. Procédé selon la revendication 2, dans lequel les fenêtres chevauchantes successives sont déplacées les unes par rapport aux autres de 5 à 15 secondes et font de 20 secondes à 1 minute de long, de préférence, sont déplacées les unes par rapport aux autres de 5 secondes et font 30 secondes de long.

4. Procédé selon l'une quelconque des revendications précédentes, comprenant en outre l'étape consistant à sous-échantillonner (14) et/ou redresser (16) le photopléthysmogramme avant modelage.

5. Procédé selon l'une quelconque des revendications précédentes, comprenant en outre l'étape consistant à effectuer un filtrage passe-bas (12) du photopléthysmogramme avant modelage.

6. Procédé selon l'une quelconque des revendications précédentes, comprenant en outre l'étape consistant à identifier en tant que pôle représentant le rythme respiratoire, un pôle (BR) ayant une grandeur supérieure à un seuil prédéterminé.

7. Procédé selon l'une quelconque des revendications précédentes, comprenant en outre l'étape consistant à identifier en tant que pôle représentant le rythme respiratoire, un pôle (BR) correspondant à une plage acceptable prédéfinie de rythmes respiratoires.

8. Procédé selon l'une quelconque des revendications précédentes, dans lequel l'ordre du modèle est de 7 à 13.

9. Procédé selon l'une quelconque des revendications précédentes, dans lequel l'ordre du modèle est 9, 10 ou 11.

10. Procédé selon l'une quelconque des revendications précédentes, comprenant en outre l'étape consistant à filtrer (22) les mesures de rythme respiratoire pour la pluralité de périodes de temps discrètes pour produire une mesure de rythme respiratoire lissée.

11. Procédé selon la revendication 10, dans lequel ladite étape de filtrage (22) comprend un filtrage de Kalman.

12. Procédé selon la revendication 10 ou 11, consistant en outre à rejeter les mesures qui représentent un changement supérieur à un changement prédéterminé dans le rythme respiratoire.

13. Procédé selon l'une quelconque des revendications précédentes, comprenant en outre l'étape consistant à combiner (26) la mesure du rythme respiratoire avec une mesure de rythme respiratoire provenant d'un autre capteur.

14. Procédé selon la revendication 13, dans lequel la combinaison est une combinaison pondérée avec des poids dérivés de la confiance dans les mesures.

15. Appareil destiné à mesurer le rythme respiratoire d'un sujet, comprenant une entrée (3) pour recevoir un photopléthysmogramme du sujet, un processeur de données (1) configuré pour exécuter les étapes consistant à modeler (18) chacune de la pluralité de périodes de temps discrètes du photopléthysmogramme en utilisant les modèles autorégressifs respectifs, trouver les pôles (20) des modèles autorégressifs des périodes de temps discrètes du photopléthysmogramme, et calculer (20) à partir des pôles de chaque période le rythme respiratoire pour chaque période de temps comme défini selon l'une quelconque des revendications 1 à 14.

PPG INPUT
3

PROCESSOR

1

OPTIONAL
OTHER BR
INPUT

4

DISPLAY

2

FIGURE 1

FIGURE 2

FIGURE 3

(a)

(a) Raw PPG

(b)

(b) Downsampled PPG

(c)

(c) Detrended downsampled PPG

Time (s)

# FIGURE 3(d)

BR
(d) AR poles

# FIGURE 4

(a) raw PPG

(b) filtered

(c) downsampled

(d) detrended

14

# FIGURE 4(e)

(e) AR poles

BR

Imaginary

0

0
Real

FIGURE 5(a)

(a)

# FIGURE 5(b)

**EP 2 173 242 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 10021438 A
- WO 2004075746 A2
- WO 03071938 A1
- WO 03051198 A1
- WO 03051198 A